# EUROPEAN PATENT SPECIFICATION

(11) **EP 1 364 036 B1**
(45) Date of publication and mention of the grant of the patent: **10.09.2008**
(21) Application number: 02722036.7
(22) Date of filing: 03.01.2002
(51) Int. Cl.: C12N 15/82, A01H 5/00

(54) **STRESS RESISTANCE AS A SELECTABLE MARKER FOR TRANSGENIC PLANTS**
STRESS-RESISTENZ ALS SELEKTIERBARER MARKER IN TRANSGENEN PFLANZEN
RESISTANCE AU STRESS COMME MARQUEUR SELECTABLE DANS DES PLANTES TRANSGENIQUES

(30) Priority: 04.01.2001 GB 0100105
(43) Date of publication of application: 26.11.2003
(73) Proprietor: Vlaams Interuniversitair Instituut voor Biotechnologie v.z.w. (VIB), 9052 Gent (BE)
(72) Inventor: THEVELEIN, Johan, B-3001 Heverlee (BE); LEYMAN, Barbara, B-9000 Gent (BE); VAN DIJCK, Patrick, B-3217 Zichem (BE); AVONCE, Nelson, C Pedregal 29, Jiutepec, Morelos c.p. 62550 (MX); ITURRIAGA, Gabriel, Cuernavaca, Morelos 62180 (MX)
(74) Representative: Bird, Ariane
(86) International application number: PCT/EP2002/000818
(87) International publication number: WO 2002/072849

(56) References cited:
- WO-A-00/60061
- WO-A-95/06126
- WO-A-96/00789
- WO-A-99/14337
- WO-A-99/54489
- PANEK A.C. ET AL: 'Regulation of trehalose metabolism in Saccharomyces cerevisiae mutants during temperature shifts' BIOCHIMIE vol. 72, 1990, pages 77 - 79

## Description

This invention relates to transgenic plant and transgenic seed production. More precisely, the invention relates to an improved method of producing transgenic plants and seeds, as well as novel transgenic plants and seeds obtainable by this method, vectors and biological kits for such production.

### BACKGROUND OF THE INVENTION

Under the influence of abiotic stress conditions such as drought, cold, salinity and heat, many living organisms adjust their osmotic potential by accumulation of solutes compatible with their metabolism such as sorbitol, mannitol, glycine-betaine, proline, sucrose or trehalose. The latter compound is a non-reducing disaccharide (α-α 1,1-diglucose) which performs an osmoprotecting function under stress conditions by interacting with membranes and proteins, thus avoiding phase separation and irreversible denaturation of the said organisms. Trehalose is present in many different living organisms such as bacteria, fungi, animals and plants. In most of them, trehalose is synthesized in two enzymatic steps: a first step from glucose-6-phosphate and uridine diphosphoglucose into trehalose-6-phosphate is catalyzed by trehalose-6-phosphate synthase (hereinafter referred as TPS, expressed by the *TPS 1* gene), then a second step from trehalose-6-phosphate into trehalose is catalyzed by trehalose-6-phosphate phosphatase (hereinafter referred as TPP, expressed by the *TPS 2* gene).

The yeast *Saccharomyces cerevisiae tps1Δ* mutant does not synthesize trehalose and is unable to grow in glucose as a unique carbon source, according to Van Aelst et al. in Mol. Microbiol. (1993) 8:927-943. This phenotype is due to an unrestricted influx of glucose into glycolysis which leads to an exhaustion of intracellular adenosine 5' triphosphate (hereinafter ATP) and inorganic phosphates. Experimental evidence from Thevelein et a!- in Trends Biochem. Science (1995) 2:410-418 suggests that trehalose-6-phosphate controls hexokinase (hereinafter referred as HXK) II, therefore the absence of this intermediate leads to high HXK II activity and hence to a metabolic overload of glucose-6-phosphate. Additionally, sugar influx could also be regulated by the TPS1 protein *per se,* according to Van Vaeck et al. in Biochem. J. (2001) 353:157-162.

Both the TPS and TPP enzymes are encoded by separate genes in yeasts such as *E. coli, Saccharomyces cerevisiae* and in plants such as *Arabidopsis thaliana* and *Selaginella lepidophylla.* In the two former species, the TPS enzyme is encoded as a 56 kD polypeptide whereas in plants it is encoded as a 100 to 109 kD protein. There is a strong similarity in protein sequence in the common region among all these TPS enzymes, except for the N- and C-terminal parts present in plant TPS proteins, as disclosed by Blázquez et al. in Plant J. (1998) 13:685-689 and Zentella et al. in Plant Physiol. (1999) 119:1473-1482. International Patent Application published as WO 00/22141 describes a method for preparing a eukaryotic organism, such as plant, animal or fungi, showing constitutive, inducible and/or organ-specific expression of a specifically modified *TPS* gene, comprising the steps of:
(a) providing a *TPS* gene,
(b) designing a suitable modification to the *TPS* gene by aligning the gene with the corresponding gene of yeast and establishing which part of the gene extends beyond the 5' terminus of the yeast gene,
(c) deleting or inactivating a part of the N-terminal region of the *TPS* gene in order to increase TPS activity,
(d) cloning the modified gene into an expression vector under the control of a constitutive, inducible and/or organ-specific promoter,
(e) transforming a plant cell or tissue with the obtained expression vector,
(f) regenerating a complete plant from the transformed plant cell or tissue.
The said document also describes plants having increased stress tolerance and harbouring in their genome such a specifically modified *TPS* gene.

There is substantial evidence that HXK is a major component in sugar-sensing in yeast and plants, according to Sheen et al. in Curr. Op. Plant Biol. (1999) 2:410-418. In the former organisms, an *hxk2*Δ mutant is able to grow normally in high-glucose media, moreover the double mutant *hxk2*Δ*tps1*Δ restores the inability of *tps1*Δ mutant to grow in glucose suggesting that HXKII is upstream in this pathway. Transgenic *Arabidopsis thaliana* (abbreviation: At) plants expressing AtHXK1 or AtHXK2 in antisense showed a glucose-insensitive phenotype, i.e. germination, development and greening proceeds in a high-glucose medium containing 6% (weight per volume) glucose, whereas wild-type seeds have arrested growth and albino appearance, in part as a result of glucose-repression of photosynthetic genes encoding certain proteins, according to Jang et al. in Plant Cell (1997) 9:5-19. In contrast, over-expression of *AtHXK* genes in *Arabidopsis thaliana* causes a glucose-hypersensitive phenotype which is more severe than wild-type when grown in high-glucose media.

Presently known markers for the selection of transformed plants belong to one of the following major classes, all of them having a number of drawbacks.

Antibiotic resistance markers as known as screening tools. Concern has been expressed however that these resistance markers might spread by horizontal gene transfer to bacterial pathogens in the human gut or elsewhere in the environment. Although this has never been demonstrated to occur in practice, the huge problems with increasing antibiotic resistance in microbial pathogens serve as a strong reminder that in the past certain possible problems have been overlooked and that this could now also be true with the introduction of transgenic plants.

Similar criticisms have been raised against other types of selective markers, such as herbicide resistance genes, which could spread in the environment to weeds and make the latter more difficult to eradicate. The antibiotic or herbicide resistance markers are only needed for the construction of the transgenic plant, and they remain afterwards in the plant as entirely useless entities. Hence, replacement of antibiotic resistance markers by other types of markers does not raise any fundamental problem for the development of transgenic plants. Therefore, although the possible dangers associated with the use of antibiotic resistance markers might be purely theoretical, they play an important role in criticisms against the use of genetically-modified organisms. The United Nation's Food and Agriculture Organization and the World Health Organization, in a recent report (http://www.who.int/fsf/GMfood/FAO-WHO_Consultation_report_2000.pdf), came to the conclusion that no evidence exists that markers currently in use involve a risk to human or domestic animal health. However, the said report adds that the possibility of transfer and expression of these genes is a risk that warrants their avoidance in the genome of widely disseminated genetically modified plants. Moreover, a recent European Commission proposal (July 2000) suggests a rule banning all antibiotic resistance genes in genetically modified crops. Hence, the use of antibiotic resistance markers is clearly no longer a method of choice for the construction of transgenic plants for use in foodstuffs.

A second class of markers involves excisable markers. Removal of the antibiotic resistance marker can be achieved with the so-called Cre-lox system disclosed by Yoder et al. in Biotechnology (1994) 12: 263-267 or through recombination within the chloroplast genome, such as disclosed by lamtham et al. in Nature Biotechnology (2000) 18:1172-1176. In the former case, the antibiotic resistance marker is introduced between two lox repeats and, upon induction of Cre recombinase using an inducible promoter, the lox sites recombine causing the marker to be excised. This approach suffers from the problem that the absence of the marker in the whole plant still has to be demonstrated convincingly. Hence, it appears preferable to completely eliminate the use of antibiotic resistance markers for the construction of food-grade transgenic plants.

Similar criticisms can be made to marker removal by recombination in plastids, which also presents additional complications. Because of the large number of plastids in a cell, thousands of copies of a marker gene are usually present in a cell. Removal of a marker gene therefore is usually not complete, therefore heteroplasmic plants containing both marker-free plastids and marker-containing plastids are necessarily produced. Generation of marker-free plants requires cytoplasmic plastid sorting during consecutive cell divisions and sub-culturing of the plants from homoplastic egg cells. It is difficult to ensure that during growth and sub-culturing of the plants, no inactive forms of the marker resistance gene have been generated which could be passed to other organisms and become reactivated by mutation or recombination. Although the absence of marker genes can be demonstrated convincingly by using Southern hybridisation, polymerase chain reaction (PCR) technology and proper controls, this method will always suffer from the fact that the marker genes have been present at some point in the process and may have resulted in yet unknown problems.

Another system makes use of the *Escherichia coli* mannose-6-phosphate isomerase gene as a marker, as disclosed by Miles et al. in Gene (1984) 32:41-48. Mannose is transported into plant cells by hexose carriers and phosphorylated by plant HXK into mannose-6-phosphate but cannot be further metabolised because of the lack of a plant mannose-6-phosphate isomerase. As a result, plant cells cannot use mannose as a carbon substrate for growth. Transformation by means of the mannose-6-phosphate isomerase gene renders transgenic plant cells able to grow on mannose as a carbon substrate. A drawback of this method is the use of a bacterial gene from an opportunistic pathogenic bacterium for the generation of the transgenic plants. Another limitation of this system is that certain plants seem to be able to use mannose as efficiently as other sugars according to Stoop et al. in Plant Physiology (1993) 103:1001-1008.

Therefore, there is a need in the art for markers for plant transformation which do not encode for antibiotic or biocide resistance markers. There is also a need in the art for markers for plant transformation which integrate at a predetermined place in the plant genome. In addition there is a need in the art for markers for plant transformation based on plant genes derived from the very same plant to be transformed or based on genes which have an enzymatic activity identical to that of a gene present in the plant to be transformed.

### SUMMARY OF THE INVENTION

The present invention provides novel markers for the selection of transgenic plants. These selectable markers result in the over-expression of a trehalose phosphate synthase gene which renders the transgenic plant significantly more resistant to one or more environmental and/or artificial stress factors. The markers involved in this invention preferably are trehalose phosphate synthase genes which, after over-expression in a plant, do not result in the accumulation of toxic compounds and/or do not change the morphology or development of the plant wherein the said gene is expressed.

The markers involved in this invention preferably are trehalose phosphate synthase genes obtained from the very same species as the plant which is to be transformed. However, genes having a similar biochemical function but derived from another plant species, or from a yeast, can also serve as a marker in accordance with the present invention. The markers of this invention can be suitably inserted into the genome of the transgenic plant of interest, and they have the useful capacity to make the transgenic plant more resistant to one or more artificial or environmental stress factors or conditions mimicking such stresses such as drought (dehydration), water-logging, heat, cold, frost, osmotic stress, salinity, light, low or excess nutrients or trace elements, pressure and the like.

The invention more specifically relates to the use of trehalose phosphate synthase as a selectable marker rendering the transgenic plants more resistant to environmental stress such as drought, osmotic stress and low phosphate concentrations.

According to the invention, the selection of transgenic plants is performed with a method which does not rely on the selection of antibiotic resistance or biocide resistance of a transgenic plant. The invention further includes vectors and biological kits comprising trehalose phosphate synthase genes that can be targeted into the genome of a plant. The invention also relates to transgenic plants and seeds, tissues, organs and cell cultures thereof obtainable and/or obtained by means of the selectable markers of this invention.

### BRIEF DESCRIPTION OF THE DRAWINGS

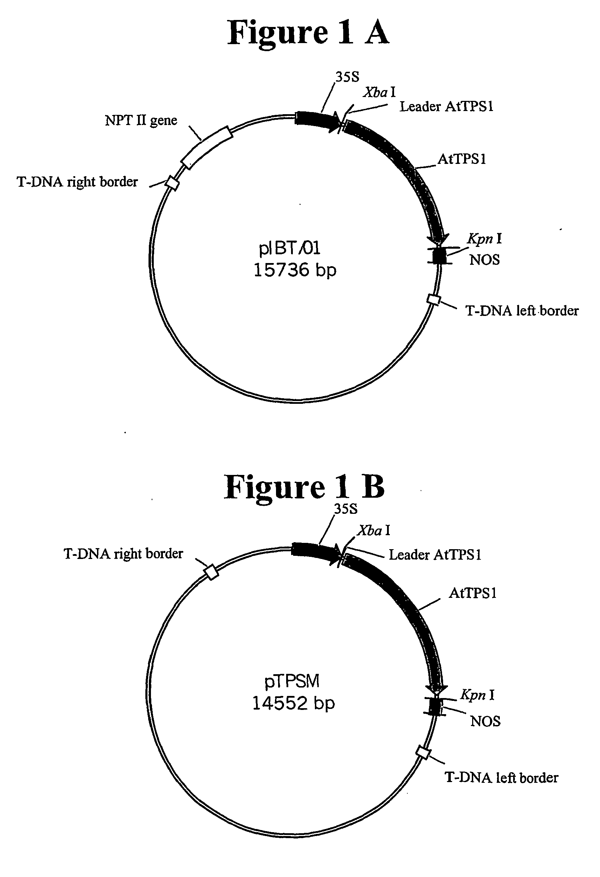

Figure 1 shows two binary vectors suitable for *Agrobacterium tumefaciens* mediated transformation of plants: plBT/01 contains *35SAtTPS1* and *Nptll* in the T-DNA (fig. 1A) and pTPSM contains *35SAtTPS1* as sole selection marker in the T-DNA (fig. 1B).

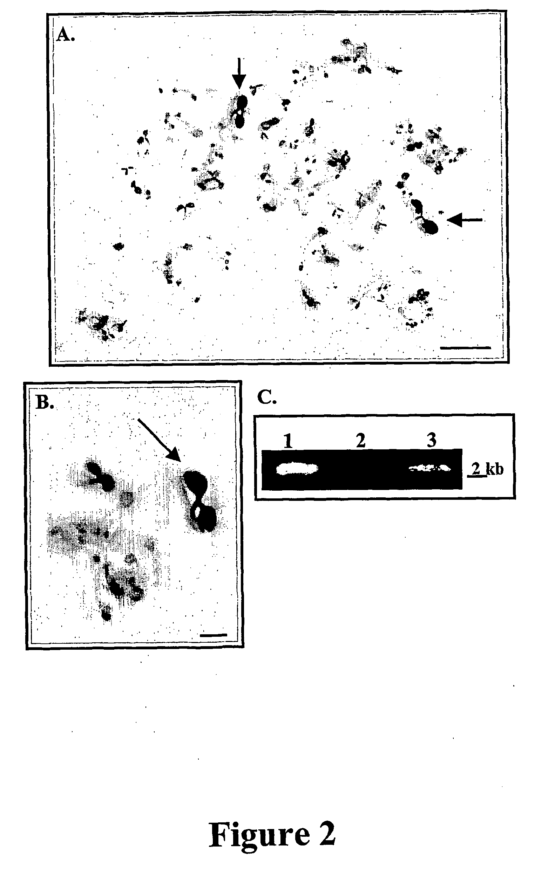

Figure 2 shows a mixture of transgenic *Arabidopsis thaliana* plants overexpressing *AtTPS1* with wild-type *Arabidopsis thaliana* seedlings, arrows indicating selected seedlings and the bar at bottom representing 5 mm (fig. 2A and 2B); fig. 2C shows a PCR product of plant genomic extracts using 35S forward and *AtTPS1* reverse primers, wherein lane 1 corresponds to the homozygous control of *35SAtTPS1,* lane 2 corresponds to a wild-type extract and lane 3 corresponds to a selected transgenic plant.

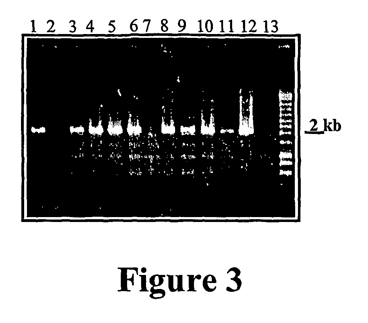

Figure 3 shows a PCR analysis of extracts from eleven *Arabidopsis thaliana* seedlings of the invention (lanes 1 to 11) selected on 6% glucose. Primers were 35S forward and *AtTPS1* reverse. Lane 12 corresponds to an extract from an *AtTPS1* overexpressing plant and lane 13 corresponds to the wild-type extract.

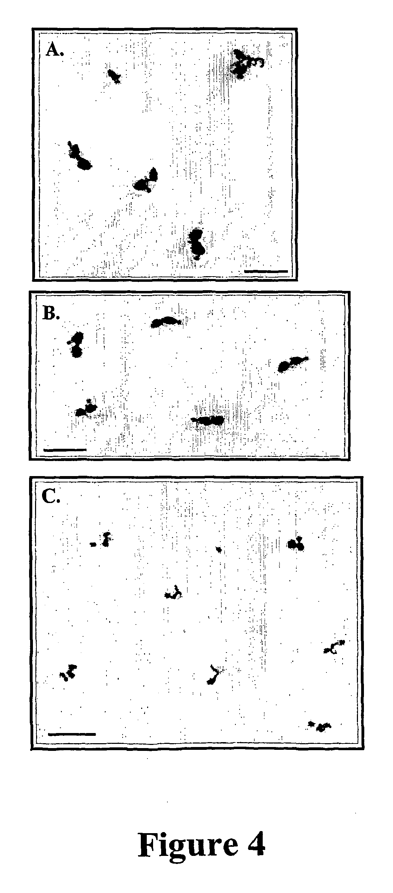

Figure 4 shows wild-type and *35SAtTPS1* overexpressing *Nicotiana tabacum* seedlings grown for 12 days on 1 x MS containing 6% glucose, from two independent transgenic lines: Nt(35SAtTPS1).05 (figure 4A) and Nt(35SAtTPS1).19 (figure 4B). Seedlings of wild type Nt are shown in figure 4C.

### DEFINITIONS

The term " homologue ", as used herein, refers to a protein sequence in which one or more amino-acids are substituted, deleted or mutated, and wherein the level of similarity with the wild-type protein is at least 80%, preferably at least 85% and more preferably at least 90%.

The term " artificial stress conditions ", as used herein, refers to stress conditions which mimick or repeat environmental stress conditions which may be applicable to the plant of interest. This specifically excludes antibiotic or biocide resistance.

### DETAILED DESCRIPTION OF THE INVENTION

The present inventors have determined, in experiments where ten independent transgenic lines were analyzed for each construct of following table 1, that an increase in trehalose content is observed when certain truncated alleles of plant TPS (ΔNAtTPS1 and ΔNSITPS1 hereunder refer to a truncation of the N-terminal region corresponding respectively to 100 or 88 amino acids in *Arabidopsis thaliana* (At) and in *Selaginella lepidophylla)* are expressed in transgenic plants. Table 1 hereunder indicates the content of trehalose in transgenic At plants. It can be seen that At plants over-expressing full-length *AtTPS1* gene are closely similar or indistinguishable from wild-type. Additionally, the observed gene-dependent gradient of morphological alterations (Δ*NSITPS1*>Δ*NAtTPS1*>*SITPS1*>*AtTPS1*) has a tight correlation with the levels of trehalose found in transgenic lines of each gene construct.

**Table 1**

| construct | Trehalose µg/g | |
|---|---|---|
| | -validomycin | +validomycin |
| AtTPS1 | 99 - 261 | 178 - 358 |
| ΔNAtTPS1 | 133 - 934 | 225 - 5974 |
| SITPS1 | 122 - 405 | 373 - 8586 |
| ΔNSITPS1 | 229 - 3645 | 369 - 53164 |
| Antisense AtTPS1 | 39 - 63 | 97-146 |
| Wild type | 27 - 47 | 36 - 77 |

In one embodiment, the invention relates to trehalose phosphate synthase proteins that, when over-expressed in a plant, increase the resistance or tolerance of the said plant to environmental stress factors such as temperature (heat or cold), osmotic pressure (drought, salinity) and the like, as well as artificial stress conditions. The corresponding genes are thus used as selectable markers for the transformation of a plant with a foreign gene. Preferably the trehalose phosphate synthase stress resistance genes, after over-expression, do not result in a phenotypic effect. However differences in phenotype can be tolerated when the transformed plants after harvesting are processed in such a way that the modified phenotype is not noticed anymore. The stress resistance gene being used as a selectable marker may be obtained from the same plant species as the plant species to be produced or transformed. However genes from other plant species, e.g. which render the plant more resistant after over-expression, can be used as well. Prokaryotic genes such as bacterial genes, whether or not having a functional homologue in higher organisms such as plants, are not within the scope of the present invention. Preferably the stress tolerance gene to be used as a selectable marker codes for the non-truncated trehalose phosphate synthase, however many other nucleotide sequences fall within the scope of this invention as explained hereinafter. In another embodiment, this invention relates to vectors and biological kits for the production or transformation of plants containing a nucleotide sequence coding for a trehalose phosphate synthase stress resistance protein. Preferably, these vectors are for *Agrobacterium tumefaciens* transfection. Transfection methods, as well as other applicable biolistic methods, are well known to those skilled in the art and are described for instance by E. Galun in *The manufacture of Medical and Health Products by Transgenic Plants* (Imperial College Press, 2001).

Very specifically, this invention relates to the use of a homologous trehalose-6-phosphate synthase gene or a N-terminally truncated fragment thereof as a selectable marker for plant transformation. This invention also relates to the use of a heterologous wild-type trehalose-6-phosphate synthase gene or a fragment thereof as a selectable marker for plant transformation. The invention also relates to the use of the N-terminus of a homologous or heterologous trehalose-6-phosphate synthase gene as a selectable marker for plant transformation.

According to one embodiment, the present invention relates to producing a transgenic plant or transgenic plant cell having increased stress resistance under environmental or artificial stress conditions, by a method comprising the steps of:
a) transforming part of a plant or part of a plant cell population with a nucleotide sequence comprising (i) a first nucleotide sequence coding for a trehalose phosphate synthase or a homologue thereof, or a catalytically active part of the said protein, the said sequence being operably linked to a promoter, and (ii) one or more restriction sites comprising a second nucleotide sequence,
b) applying environmental or artificial stress conditions to the transformed plant or plant cell population obtained in step (a), or its progeny, and
c) selecting, from the transformed plant or plant cell population placed under the environmental or artificial stress conditions of step (b), that part of the transformed plant or plant cell population which exhibits an increased stress resistance under environmental or artificial stress conditions.

Preferably, the said trehalose phosphate synthase or homologue or catalytically active part thereof, is selected to be a protein which does not alter the phenotype of the plant being transformed in respect of properties or characteristics other than stress resistance.

The second nucleotide sequence to be used in the above method is not a critical parameter of the invention. For instance, it may be a nucleotide sequence coding for an antibody, an antibody fragment (such as a single-chain variable fragment, a F_{ab} fragment, a F_{(ab')2} fragment, or a complementary determining region), an antigen, a therapeutic protein, an enzyme inhibitor, a cytokine, a growth factor, a peptide hormone, a protease, an enzyme or an enzymatic product thereof.

As disclosed herein-above, the presence of the first nucleotide sequence acts as a marker for (i.e. is indicative of) the presence of the second nucleotide sequence in the transgenic plant or transgenic plant cell having increased stress resistance to be produced. Preferably, the first nucleotide sequence and the second nucleotide sequence are integrated into the genome of the said transgenic plant or transgenic plant cell having increased stress resistance.

When a catalytically active part of a trehalose phosphate synthase protein is used in the method of the invention, this may be the N-terminally truncated fragment or the C-terminally truncated fragment of the said protein. According to a preferred embodiment of the invention, the said eukaryotic stress resistance protein is an enzyme catalysing the production of a sugar phosphate, a glycerol phosphate or a sugar-alcohol phosphate, such as trehalose-6-phosphate synthase (TPS). More preferably the first nucleotide sequence used in the method of the invention is one coding for a non-truncated trehalose-6-phosphate synthase.

For illustrative purposes only, other examples of eukaryotic stress resistance proteins include :
- the NHX1 protein reviewed by Zhang et al. in Proc. Natl. Acad. Sci. USA (2001) 98:12832-6,
- heat-shock proteins such as reviewed by Sun et al. in Plant J. (2001) 27:407-15,
- the AVP1 protein reviewed by Gaxiola et al. in Proc. Natl. Acad. Sci. USA (2001) 98:11444-9,
- the NCED protein reviewed by luchi et al. in Plant J. (2001) 27:325-333,
- the APX1 protein reviewed by Shi et al. in Gene (2001) 273:23-7,
- the APX3 protein reviewed by Wang et al. in Plant Cell Physiol. (1999) 40:725-32,
- the BiP protein (binding protein) reviewed by Alvim et al. in Plant Physiol. (2001) 126:1042-54,
- the OASTL protein reviewed by Youssefian et al. in Plant Physiol. (2001) 126:1001-11,
- the DREB1 protein reviewed by Yamaguchi-Shinozaki et al. in Novartis Found. Symp. (2001) 236:176-86,
- the SCOF1 protein (a cold-inducible zinc finger protein) reviewed by Kim et al. in Plant J. (2001) 25:247-59,
- the GST/GPX protein reviewed by Roxas et al. in Plant Cell Physiol. (2000) 41:1229-34,
- the GS2 protein reviewed by Hoshida et al. in Plant Mol. Biol. (2000) 43:103-11,
- the HAL1 protein reviewed by Gisbert et al. in in Plant Physiol. (2000) 123:393-402,
- the PRP2 protein reviewed by Winicov in Planta (2000) 210:416-22, and
- the SAT protein reviewed by Blaszczyk et al. in Plant J. (1999) 20:237-243.

The first nucleotide sequence may also code for a chimeric protein combining domains from the stress resistance trehalose phosphate synthase proteins from two or more different plant species.

For a successful performance of the method of the invention, for instance when the eukaryotic stress resistance protein is an enzyme catalysing the production of a sugar phosphate, a glycerol phosphate or a sugar-alcohol phosphate, more particularly trehalose phosphate synthase, the stress conditions applied in step (b) of the method preferably are artificial stress conditions induced by contacting the transformed plant or plant cell population obtained in step (a), or its progeny, with a medium containing a high content, i.e. from about 3 to 8% by weight and more preferably from about 4 to 7% by weight, of a sugar, a sugar-alcohol or a glycerol such as for instance sucrose or saccharose.

The first nucleotide sequence used in the method of the invention may be from a plant or from a yeast. The choice of the relevant plant or yeast for this purpose is not a critical parameter of the present invention. Suitable plants for this purpose include, among others, dicotyledonous plants such as tobacco *(Nicotiana tabacum),* soybean *(Glycine max),* apple, sugarbeet, *Arabidopsis thaliana,* alfalfa, petunia, cotton, carrot, celery, cabbage, cucumber, pepper, canola, tomato, potato, lentil, flax, broccoli, bean, lettuce, oilseed rape, cauliflower, spinach, brussel sprout, artichoke, pea, okra, squash, kale, collard greens, tea, coffee and *Selaginella lepidophylla.* They also include monocotyledonous plants such as rice *Oryza sativa,* corn, barley, maize, sunflower *(Helianthus annuus),* wheat, oats, millet, sorghum, amaranth, onion, asparagus and sugar cane. A suitable originating yeast for the first nucleotide sequence is *Saccharomyces cerevisiae.*

The promoter to which the first nucleotide sequence is operably linked is not a critical parameter of the present invention. It may be a constitutive promoter, an inducible promoter or a tissue-specific promoter, such categories being well known to those skilled in the art with particular advantages in particular cases. A preferred commercially available example of a suitable constitutive promoter is the 35S cauliflower mosaic virus (CaMV) promoter. A well known example of a plant promoter that causes expression specifically in storage organs is the patatin promoter. Examples of plant promoters that are induced by stress include for instance the LTI78 promoter (disclosed by Nordin et al. in Plant Mol. Biol. (1993) 21:641-653) and the RAB18 promoter (disclosed by Lang et al. in Plant Mol. Biol. (1992) 20:951-962).

Some aspects of promoter selection will now be discussed in more detail with reference to the specific case wherein the first nucleotide sequence codes for a non-truncated trehalose-6-phosphate synthase. Although the accumulation of trehalose at certain times (e.g. during exposure to stress or in a mature plant) and in certain tissues (e.g. storage organs or, at appropriate times, frost-sensitive tissues) is expected to be beneficial, or at least harmless to a plant, there is also a possibility that at other times and in other tissues the accumulation of trehalose may be harmful to a plant, as disclosed by Veluthambi et al. in Plant Physiol. (1981) 68:1369-1374. It may therefore be advantageous to use a plant promoter that does not permit full expression of the gene causing trehalose synthesis until the plant is mature or encounters environmental conditions, including drought and low temperature, in which the benefits of trehalose outweigh its possible disadvantages to the plant. Several examples of such non-constitutive plant promoters are known to those skilled in the art, including the ribulose-1,5-bisphosphate carboxylase (Rubisco) promoter disclosed by Krebbers et al. in Plant Mol. Biol. (1988) 11:745-759. On the other hand, use of stress-induced promoters will prevent the accumulation of trehalose until it is needed. In plants able to grow whilst containing trehalose, the invention may be performed without resource to a stress-induced promoter, however the use of stress-induced promoters to prevent trehalose production until it is needed has the additional advantage of avoiding the yield penalty that would otherwise result from the diversion of photosynthetic capacity to trehalose synthesis.

There are several ways, all well known to those skilled in the art, of performing the transforming step (a) of the method of the invention, including the practice of genetic transformation mediated by *Agrobacterium tumefaciens* and the method of biolistic transformation, both being shortly described by E. Galun (cited *supra).*

The plant or plant cell population being transformed during step (a) of the method of the invention is not a critical parameter of the present invention. It may be for instance a dicotyledonous plant such as tobacco *(Nicotiana tabacum),* soybean *(Glycine max),* apple, sugarbeet, *Arabidopsis thaliana* alfalfa, petunia, cotton, carrot, celery, cabbage, cucumber, pepper, canola, tomato, potato, lentil, flax, broccoli, bean, lettuce, oilseed rape, cauliflower, spinach, brussel sprout, artichoke, pea, okra, squash, kale, collard greens, tea, coffee and *Selaginella lepidophylla.* It may also be a monocotyledonous plant such as rice *Oryza sativa,* com, barley, maize, sunflower (*Helianthus annuus),* wheat, oats, millet, sorghum, amaranth, onion, asparagus or sugar cane.

According to another embodiment, the present invention relates to a transgenic plant or part thereof, a transgenic plant seed or a transgenic plant cell being obtainable by the production method described herein-before. In terms of product, this may be broadly described as a transgenic plant or part thereof, a transgenic plant seed or a transgenic plant cell having, integrated into its genome, (i) a first nucleotide sequence coding for a trehalose phosphate synthase protein, or a homologue thereof, or a catalytically active part of the said protein, the said sequence being operably linked to a promoter, and (ii) a second nucleotide sequence. Its various components, i.e. the first nucleotide sequence coding for a trehalose phosphate synthase protein (or a homologue or a catalytically active part thereof), the promoter and the second nucleotide sequence, have been described in details with respect to the method of production, hence there is no need to repeat their meaning here.

In yet another embodiment, the method of production of this invention may make use of a vector which may be broadly described as comprising (i) a first nucleotide sequence coding for a trehalose phosphate synthase protein, or a homologue thereof, or a catalytically active part of the said protein, the said sequence being operably linked to a promoter, and (ii) one or more restriction sites for the insertion of a second nucleotide sequence. In a further embodiment, the method of production of this invention may make use of a specific biological kit which may be broadly described as comprising (1) a vector such as previously described and (2) one or more nucleotide sequences from which the insertion of the first nucleotide sequence and/or the second nucleotide sequence into the genome of the transgenic plant or transgenic plant cell can be evaluated. Such evaluation may be performed by methods such as Southern blot hybridization with vector sequences or by PCR primers, e.g. flanking restriction sites wherein the transgene was cloned or by primers in or adjacent to the introduced stress resistance gene, for instance SEQ.ID.No.1 or SEQ.ID.No2 disclosed in example 4 herein-after. The various terms used in the definition of the vector or the biological kit of this invention are used herein with the same meanings as in the part of the specification devoted to the method of production.

As the skilled person will readily understand, the present invention shows a significant advantage in terms of an environmentally friendly technology avoiding the use of antibiotic or biocide resistance markers.

The present invention will now be explained and illustrated with reference to a limited number of examples which however should not be construed as limiting the scope of the invention.

### EXAMPLE 1 - over-expression in A. thaliana of the AtTPS1 N-terminal region

Several *A. thaliana* transgenic lines over-expressing Δ*NSITPS1,* Δ*NAtTPS1, SITPS1* or *AtTPS1* were germinated in the presence of high-glucose media (i.e. 6% by weight). Additionally, several *AtTPS1* antisense transgenic lines were generated and tested accordingly. Plants were germinated at constant light and 23°C. After one week a remarkable difference was observed between wild-type and antisense plants, on the one hand, and over-expression of plant *TPS1* alleles on the other hand. The latter plants have a normal germination, hypocotyl elongation, and greening and expansion of cotyledonary leaves in high-glucose media. In contrast, wild-type plants looked white and much smaller. Transgenic lines expressing antisense *AtTPS1* were even smaller and germinated at lower rate than wild-type. In normal media (i.e. 1% by weight glucose) all plants harboring the various plant *TPS1* constructs looked normal and showed no significant difference among them.

The glucose insensitive phenotype was most remarkable in plants over-expressing the *AtTPS1* alelle, suggesting that the presence of the N-terminal region when the over-expressed *TPS1* gene belongs to the homologous plant, is a key element in the sugar-sensing properties of the plant TPS1 protein. This glucose insensitive phenotype is not necessarily associated with the synthesis of trehalose, i.e. there may be no correlation between the accumulation of higher levels of trehalose and the severity of the glucose insensitive phenotype. One possible explanation may be that over-expression in *A*. *thaliana* of the AtTPS1 N-terminal region as such mimics part of the glucose insensitive phenotype. These data thus strongly suggest that the TPS1 protein as such has a regulatory role in controlling sugar-sensing in plants and that an important part of this phenomenon is accomplished by its N-terminal region.

### EXAMPLE 2 - plant TPS confers adaptation to growth in low-phosphate media

The over-expression of TPS genes in plants leads to trehalose accumulation as a result of trehalose-6-phosphate breakdown by an endogenous phosphatase. Engineering this metabolic route yields inorganic phosphate as a by-product. Therefore, we tested the behavior of transgenic plants harboring various plant TPS constructs in high or low phosphate media. In the following experiments, high phosphate media stands for normal MS media (i.e. having 1 mM Na₂PO₄) whereas low phosphate media has only 1 µM Na₂PO₄ as reported by López-Bucio et al. in Nature Biotechnol. (2000) 18:450-453. In order to observe growth differences, plants were grown for three weeks with 16 hours day-length at 23°C. Transgenic plants over-expressing plant *TPS1* homologues were able to grow in low phosphate media at a normal rate without any obvious morphological alteration, whereas wild-type plants showed severe growth retardation (size decreased to about 25-35% of the normal size) and an increasing brownish color characteristic of phosphate deprivation according to Raghatoma in Current Op. Plant Biol. (2000) 3:182-187. Transgenic lines expressing antisense *AtTPS1* gene were even more sensitive to low phosphate than wild-type.

As in example 1, transgenic lines over-expressing *AtTPS1* showed the best performance in low-phosphate media. Therefore from these data it may be concluded that plant TPS1 protein preferably containing the intact N-terminal is somehow involved in inorganic phosphate (Pi) accumulation by a regulatory mechanism which is not necessarily linked to the liberation of Pi by trehalose-6-phosphate dephosphorylation.

### EXAMPLE 3 - construction of a vector with a selectable marker

In order to use the *AtTPS1* gene as a marker on a binary vector, a pTPSM construct was made. The pTPSM vector is a derivative of pBlN19 lacking the kanamycine resistant gene (*Nptll*). Like plBT/01, pTPSM contains the *AtTPS1* cDNA under control of the 35S promoter within the T-DNA, as shown in figure 1. The pTMS vector was made in several steps: pBN35 is a pBlN19 vector containing the 35S promoter and Nos terminator without inserted cDNA inbetween. The *Nptll* gene was eliminated from pBN35 by cutting the vector with *Apa*l and *Nhe*l and after T4 polymerase activity re-ligating the rest of the vector resulting in pEBN35. AtTPS1 was then cut from the plBT/01 vector using *Kpn*l and *Xba*l double digestion and, after first ligating in pBLuscript, AtTPS1 was ligated between the *Kpn*l and *Xba*l site of pEBN35S, resulting in the pTPSM construct. This vector may still be improved by adding more unique restriction sites in the T-DNA in order to ligate a gene of specific interest.

### EXAMPLE 4 - selection of transgenic plants

*Agrobacterium tumefaciens* C58C1 was transformed with plBT/01 or pTPSM by electroporation. *Arabidopsis* plants were grown until flowering and transformed using the flower dip method disclosed by Desfeux et al. in Plant Phys. (2000) 23:895. Mature seeds of the transformed plants were collected, dried at 23°C for two weeks and then stored at 4°C.

In order to select the transgenic seeds from a pool of non-transformed seeds, they were first vapour-sterilised, then sterile water was added to the seeds which were incubated under constant light at 4°C for 24 hours. Growth medium consisted of 1 x Murashige and Skoog salts and Vitamin mixture (commercially available from Life Technologies); 2.5 mM MES pH 5.7 adjusted with KOH; 0.8% phytagar; and 6% glucose added prior to pouring plates (glucose was autoclaved separately from the rest of the medium). Seeds were dispersed on plates in low density (i.e. maximum 2000 seeds per plate of ∅ 145 mm) using 2-3 ml H₂O (0.1 % agar or agarose masked the glucose sensitivity of wild-type seeds). filter paper (Whatman No.1) can be used to prevent seeds from clustering. Isolated seedlings sensed the same level of glucose, accentuating the difference between transgenics and wild-type. Plant growth conditions were 22°C and 24 hours light and Petri dishes were closed in order to maintain very high humidity. Seeds were incubated under such growth conditions for 5 to 7 days.

Selected plants were grown for 2 to 3 weeks in soil. Leaves were used to prepare genomic DNA, PCR analysis being performed by using the following primers, annealing temperature being set at 56°C :
35S forward: AAGAAGACGTTCCAACCACG [SEQ ID NO:1]
*AtTPS1* reverse: CGCTCAGAACAACTATGGTT [SEQ ID NO:2]

### EXAMPLE 5 - recovery of transformed plants in a large population of seeds.

In order to optimise conditions for the selection of transgenic *Arabidopsis* plants using the *AtTPS1* selective marker, we initially made a mixture containing 1000 times more wild-type seeds than the already available *35SAtTPS1* over-expressing lines which have been transformed with the plBT/01vector. The seed blend was sterilised and vernalized for 24 hours at 4°C with constant light. 1x Murashige and Skoog based medium was enriched with 6% glucose. The seed mixture was sown in a concentration of 2000 seeds per plate (∅ 145mm) using water. Plants were germinated at 22°C with constant light. 6 days after sowing, photographs were taken of a fraction of the plants (Fig. 2A and 2B). Under these conditions, wild-type *Arabidopsis* seedlings remained white and small whereas 35SAtTPS1 transformed seedlings had a healthy appearance with green and larger cotyledons.

The large seedling as shown in figure 2 was tested for the presence of the *35SAtTPS1* cassette by polymerase chain reaction (PCR), using genomic DNA as a template. The forward primer located near the end of the 35S promoter and the reverse primer in a specific region of the *AtTPS1* sequence lead to the amplification of a 2132 bp product. A PCR product with the predicted size was obtained when DNA of the selected seedling was used as template as shown in figure 2C, confirming that the selected seedling indeed carried the *35SAtTPS1* gene in its genome.

In order to evaluate the reliability of the selection performed, the experiment was scaled-up. Approximately 4000 seeds were sown on a 6% glucose containing MS medium. Six days later, the larger seedlings with green cotyledons were selected amongst the pool of white and small seedlings. PCR analysis show that more than 80% of the selected seedlings contained the *35SAtTPS1* cassette as shown in figure 3.

### EXAMPLE 6 - USE OF AN ARABIDOPSIS TPS GENE AS A SELECTABLE MARKER FOR TRANSFORMED TOBACCO PLANTS

*Nicotiana tabacum* seeds carrying the *35SAtTPS1* cDNA in their genome germinate faster in high-glucose medium and hence are less sensitive to glucose than wild-type seeds, as shown in table 2 hereunder indicating the germination percentage on 6% by weight glucose-containing medium respectively 10, 11 and 12 days after sowing. Wild-type seedlings were not whiter, but definitely smaller than transgenic plants as shown in figure 4. Even though a different transformation protocol may be required for *N.tabacum* in comparison with *A.thaliana,* adjustment of conditions enables to select tobacco transgenic plants on glucose as well.

**Table 2**

| | Day 10 | Day 11 | Day 12 |
|---|---|---|---|
| Nt WT | 0 | 13.6 | 63.6 |
| Nt(35SAtTPS1).05 | 23.5 | 59 | 82.4 |
| Nt(35SAtTPS1).19 | 39 | 55.5 | 77.8 |

## Claims

1. A method of obtaining a transgenic plant or transgenic plant cell comprising a second nucleotide sequence said method comprising the steps of:
a) transforming part of a plant or part of a plant cell population with a nucleotide sequence comprising (i) a first nucleotide sequence coding for a trehalose phosphate synthase, a homologue, or a catalytically active part of said trehalose phosphate synthase, said first nucleotide sequence being operably linked to a promoter, and (ii) one or more restriction sites comprising said second nucleotide sequence,
b) applying environmental or artificial stress conditions to the transformed plant or plant cell population obtained in step (a), or its progeny, and
c) selecting, from the transformed plant or plant cell population placed under the environmental or artificial stress conditions of step (b), that plant, part of the transformed plant or plant cell population which exhibits an increased stress resistance under environmental or artificial stress conditions,
wherein the artificial stress conditions exclude the use of antibiotics or biocides.

2. The method according to claim 1, wherein expression of said first nucleotide sequence does not alter the phenotype of the plant being transformed in respect of properties or characteristics other than stress resistance.

3. The method according to claim 1 or claim 2, wherein said second nucleotide sequence codes for an antibody, an antibody fragment, an antigen, a therapeutic protein, an enzyme inhibitor, a cytokine, a growth factor, a peptide hormone, a protease, an enzyme or an enzymatic product thereof.

4. The method according to any of claims 1 to 3, wherein said first nucleotide sequence and said second nucleotide sequence are integrated into the genome of said transgenic plant or transgenic plant cell having increased stress resistance.

5. The method according to any of claims 1 to 4, wherein said first nucleotide sequence encodes an N-terminally truncated fragment comprising the catalytically active part of said trehalose phosphate synthase.

6. The method according to any one of claims 1 to 5, wherein the stress conditions applied in step (b) are artificial stress conditions induced by contacting the transformed plant or plant cell population obtained in step (a), or its progeny, with a medium containing from about 3 to 8% by weight of a sugar, a sugar-alcohol or a glycerol.

7. The method according to claim 6, wherein the sugar is glucose.

8. The method according to any one of claims 1 to 7, wherein said first nucleotide sequence codes for a non-truncated trehalose-6-phosphate synthase.

9. The method according to any of claims 1 to 8, wherein said first nucleotide sequence is from a plant.

10. The method according to claim 9, wherein said first nucleotide sequence is from the same plant species as the plant species being transformed in step (a) or from a plant species different from the plant species being transformed in step (a).

11. A method according to claim 9, wherein said first nucleotide sequence is from a dicotyledonous plant.

12. A method according to claim 11, wherein said dicotyledonous plant is selected from the group consisting of tobacco (*Nicotiana tabacum*)*,* soybean *(Glycine max),* apple, sugarbeet, *Arabidopsis thaliana* alfalfa, petunia, cotton, carrot, celery, cabbage, cucumber, pepper, canola, tomato, potato, lentil, flax, broccoli, bean, lettuce, oilseed rape, cauliflower, spinach, brussel sprout, artichoke, pea, okra, squash, kale, collard greens, tea, coffee and *Selaginella lepidophylla.*

13. The method according to claim 9, wherein the first nucleotide sequence is from a monocotyledonous plant.

14. The method according to any of claims 1 to 8, wherein said first nucleotide sequence is from a yeast.

15. The method according to claim 14, wherein said yeast is *Saccharomyces cerevisiae.*

16. The method according to any of claims 1 to 15, wherein said promoter is selected from the group consisting of constitutive promoters, inducible promoters and tissue-specific promoters.

17. The method according to any one of claims 1 to 7, wherein said first nucleotide sequence codes for a chimeric protein combining different domains of said trehalose phosphate synthase from at least two different plant species.

18. A transgenic plant or part thereof, a transgenic plant seed or a transgenic plant cell obtainable by the method according to any one of claims 1 to 17, **characterized in that** said trehalose phosphate synthase, homologue, or catalytically active part thereof is the sole selectable marker transformed into said part of a plant or plant cell population.

19. Use of a homologous trehalose-6-phosphate synthase gene or an N-terminally truncated fragment thereof or a heterologous trehalose-6-phosphate synthase gene or a fragment thereof or the N-terminus of a homologous or heterologous trehalose-6-phosphate synthase gene as a selectable marker for plant transformation.

## Patentansprüche

1. Verfahren, um eine transgene Pflanze oder eine transgene Pflanzenzelle, die eine zweite Nukleotidsequenz aufweisen, zu erhalten, wobei das Verfahren die Schritte umfasst:
a) Transformieren eines Teils einer Pflanze oder eines Teils einer Pflanzenzellpopulation mit einer Nukleotidsequenz, die (i) eine erste Nukleotidsequenz aufweist, die für eine Trehalose-Phosphatsynthase, ein Homolog oder einen katalytisch aktiven Teil der Trehalose-Phosphatsynthase kodiert, wobei die erste Nukleotidsequenz funktionell an einen Promotor verbunden ist, und (ii) ein oder mehrere Restriktionsstellen, die die zweite Nukleotidsequenz aufweisen,
b) Ausüben von Umwelt- oder künstlichen Stressbedingungen auf die transformierte Pflanze oder die transformierte Pflanzenzellpopulation aus Schritt (a), oder ihre Nachkommen, und
c) Auswählen aus der transformierten Pflanzen- oder der transformierten Pflanzenzellpopulation, welche den Umwelt- oder künstlichen Stressbedingungen des Schritts (b) ausgesetzt wurde, diejenige Pflanze, den Teil der transformierten Pflanze oder der Pflanzenzellpopulation, die eine erhöhte Stressresistenz unter Umwelt- oder künstlichen Stressbedingungen aufweist,
wobei die künstlichen Stressbedingungen die Verwendung von Antibiotika oder Biociden ausschließen.

2. Verfahren nach Anspruch 1, wobei die Expression der ersten Nukleotidsequenz den Phänotyp der transformierten Pflanze in Bezug auf die Eigenschaften oder Charakteristika mit Ausnahme der Stressresistenz nicht ändert.

3. Verfahren nach Anspruch 1 oder Anspruch 2, wobei die zweite Nukleotidsequenz für einen Antikörper, ein Antikörperfragment, ein Antigen, ein therapeutisches Protein, einen Enzyminhibitor, ein Cytokin, einen Wachstumsfaktor, ein Peptidhormon, eine Protease, ein Enzym oder ein enzymatisches Produkt hiervon kodiert.

4. Verfahren nach einem der Ansprüche 1 bis 3, wobei die erste Nukleotidsequenz und die zweite Nukleotidsequenz in das Genom der transgenen Pflanze oder der transgenen Pflanzenzelle mit erhöhter Stressresistenz integriert werden.

5. Verfahren nach einem der Ansprüche 1 bis 4, wobei die erste Nukleotidsequenz ein N-terminal verkürztes Fragment kodiert, welches den katalytisch aktiven Teil der Trehalosephosphatsynthase umfasst.

6. Verfahren nach einem der Ansprüche 1 bis 5, wobei die im Schritt (b) ausgeübten Stressbedingungen künstliche Stressbedingungen sind, die durch Inkontaktbringen der transformierten Pflanze oder der transformierten Pflanzenzellpopulation aus Schritt (a), oder ihrer Nachkommen, mit einem etwa 3 bis 8 Gew.-% eines Zuckers, eines Zuckeralkohols oder eines Glycerins enthaltenden Mediums induziert werden.

7. Verfahren nach Anspruch 6, wobei der Zucker Glukose ist.

8. Verfahren nach einem der Ansprüche 1 bis 7, wobei die erste Nukleotidsequenz für eine nicht-trunkierte Trehalose-6-Phosphatsynthase kodiert.

9. Verfahren nach einem der Ansprüche 1 bis 8, wobei die erste Nukleotidsequenz aus einer Pflanze stammt.

10. Verfahren nach Anspruch 9, wobei die erste Nukleotidsequenz aus der gleichen Pflanzenspecies als die Pflanzenspecies stammt, die im Schritt (a) transformiert wurde, oder aus einer Pflanzenspecies, die sich von der Pflanzenspecies unterscheidet, die im Schritt (a) transformiert wurde.

11. Verfahren nach Anspruch 9, wobei die erste Nukleotidsequenz aus einer Dikotyledonen-Pflanze stammt.

12. Verfahren nach Anspruch 11, wobei die Dikotyledonen-Pflanze ausgewählt wird aus der Gruppe, bestehend aus Tabak (*Nicotiana tabacum*), Sojabohne (*Glycine max*), Apfel, Zuckerrübe, *Arabidopsis thaliana* alfalfa, Petunien, Baumwolle, Karotte, Sellerie, Kohl, Gurke, Pfeffer, Kanola, Tomate, Kartoffel, Linse, Flachs, Brokkoli, Bohne, Salat, Ölsaaten - Raps, Blumenkohl, Spinat, Rosenkohl, Artischocke, Erbse, Okra, Kürbis, Grünkohl, Kohlblättern, Tee, Kaffee und *Selaginella lepidophylla.*

13. Verfahren nach Anspruch 9, wobei die erste Nukleotidsequenz aus einer Monocotyledonen-Pflanze stammt.

14. Verfahren nach einem der Ansprüche 1 bis 8, wobei die erste Nukleotidsequenz aus einer Hefe stammt.

15. Verfahren nach Anspruch 14, wobei die Hefe *Saccharomyces cerevisiae* ist.

16. Verfahren nach einem der Ansprüche 1 bis 15, wobei der Promotor ausgewählt wird aus der Gruppe, bestehend aus konstitutiven Promotoren, induzierbaren Promotoren und gewebespezifischen Promotoren.

17. Verfahren nach einem der Ansprüche 1 bis 7, wobei die erste Nukleotidsequenz für ein chimäres Protein kodiert, welches verschiedene Domänen der Trehalosephosphatsynthase aus zumindest zwei verschiedenen Pflanzenspecies vereinigt.

18. Transgene Pflanze oder Teil hiervon, ein transgener Pflanzensamen oder eine transgene Pflanzenzelle, erhältlich durch das Verfahren nach einem der Ansprüche 1 bis 17, **dadurch gekennzeichnet, dass** die Trehalose-phosphatsynthase, ihr Homolog oder ein katalytisch aktiver Teil hiervon der einzig selektierbare Marker ist, der in den Teil einer Pflanzenzelle oder der Pflanzenzellenpopulation transformiert wurde.

19. Verwendung eines homologen Trehalose-6-Phosphatsynthasegens oder eines N-terminal trunkierten Fragments hiervon oder eines heterologen Trehalose-6-Phosphatsynthasegens oder eines Fragments hiervon oder des N-Terminus eines homologen oder heterologen Trehalose-6-Phosphatsynthasegens als selektierbaren Marker zur Pflanzentransformation.

## Revendications

1. Procédé d'obtention d'une plante transgénique ou d'une cellule de plante transgénique comprenant une seconde séquence de nucléotides, ledit procédé comprenant les étapes :
a) de transformation d'une partie d'une plante ou d'une partie d'une population de cellules de plante avec une séquence de nucléotides comprenant (i) une première séquence de nucléotides codant pour une tréhalose-phosphate synthase, un homologue, ou une partie catalytiquement active de ladite tréhalose-phosphate synthase, ladite première séquence de nucléotides étant opérationnellement liée à un promoteur, et (ii) un ou plusieurs sites de restriction comprenant ladite seconde séquence de nucléotides,
b) d'application de conditions de stress environnemental ou artificiel à la plante ou à la population de cellules de plante transformée obtenue dans l'étape (a), ou à sa descendance, et
c) de sélection, à partir de la plante ou de la population de cellules de plante transformée placée dans les conditions de stress environnemental ou artificiel de l'étape (b), de la plante, de la partie de la plante ou de la population de cellules de plante transformée qui présente une résistance au stress améliorée dans des conditions de stress environnemental ou artificiel,
dans lequel les conditions de stress artificiel excluent l'utilisation d'antibiotiques ou de biocides.

2. Procédé selon la revendication 1, dans lequel l'expression de ladite première séquence de nucléotides ne modifie pas le phénotype de la plante subissant la transformation quant aux propriétés ou aux caractéristiques autres que la résistance au stress.

3. Procédé selon la revendication 1 ou la revendication 2, dans lequel ladite seconde séquence de nucléotides code pour un anticorps, un fragment d'anticorps, un antigène, une protéine thérapeutique, un inhibiteur d'enzyme, une cytokine, un facteur de croissance, une hormone peptidique, une protéase, une enzyme ou un produit enzymatique de celle-ci.

4. Procédé selon l'une quelconque des revendications 1 à 3, dans lequel ladite première séquence de nucléotides et ladite seconde séquence de nucléotides sont intégrées dans le génome de ladite plante transgénique ou de ladite cellule de plante transgénique ayant une résistance au stress améliorée.

5. Procédé selon l'une quelconque des revendications 1 à 4, dans lequel ladite première séquence de nucléotides code pour un fragment tronqué à l'extrémité N-terminale comprenant la partie catalytiquement active de ladite tréhalose-phosphate synthase.

6. Procédé selon l'une quelconque des revendications 1 à 5, dans lequel les conditions de stress appliquées dans l'étape (b) sont des conditions de stress artificiel induites par la mise en contact de la plante ou de la population de cellules de plante transformée obtenue dans l'étape (a), ou de sa descendance, avec un milieu contenant entre environ 3 et 8 % en poids d'un sucre, d'un alcool glucidique ou d'un glycérol.

7. Procédé selon la revendication 6, dans lequel le sucre est le glucose.

8. Procédé selon l'une quelconque des revendications 1 à 7, dans lequel ladite première séquence de nucléotides code pour une tréhalose-6-phosphate synthase non tronquée.

9. Procédé selon l'une quelconque des revendications 1 à 8, dans lequel ladite première séquence de nucléotides provient d'une plante.

10. Procédé selon la revendication 9, dans lequel ladite première séquence de nucléotides provient de la même espèce de plante que l'espèce de la plante subissant la transformation dans l'étape (a) ou d'une espèce de plante différente de celle de la plante subissant la transformation dans l'étape (a).

11. Procédé selon la revendication 9, dans lequel ladite première séquence de nucléotides provient d'une plante dicotylédone.

12. Procédé selon la revendication 11, dans lequel ladite plante dicotylédone est choisie dans le groupe constitué par le tabac *(Nicotiana tabacum*), le soja *(Glycine max),* la pomme, la betterave à sucre, la luzerne *Arabidopsis thaliana,* le pétunia, le coton, la carotte, le céleri, le chou, le concombre, le poivre, le colza, la tomate, la pomme de terre, la lentille, le lin, le brocoli, le haricot, la laitue, la graine de colza, le chou-fleur, l'épinard, le chou de Bruxelles, l'artichaut, le pois, l'okra (gombo), la courge, le chou frisé, le chou vert, le thé, le café et *Selaginella lepidophylla* (rose de Jéricho).

13. Procédé selon la revendication 9, dans lequel la première séquence de nucléotides provient d'une plante monocotylédone.

14. Procédé selon l'une quelconque des revendications 1 à 8, dans lequel ladite première séquence de nucléotides provient d'une levure.

15. Procédé selon la revendication 14, dans lequel ladite levure est *Saccharomyces cerevisiae.*

16. Procédé selon l'une quelconque des revendications 1 à 15, dans lequel ledit promoteur est choisi dans le groupe constitué par des promoteurs constitutifs, des promoteurs inductibles et des promoteurs spécifiques de tissus.

17. Procédé selon l'une quelconque des revendications 1 à 7, dans lequel ladite première séquence de nucléotides code pour une protéine chimérique combinant différents domaines de ladite tréhalose-phosphate synthase provenant d'au moins deux espèces de plantes différentes.

18. Plante transgénique ou partie de celle-ci, graine de plante transgénique ou cellule de plante transgénique pouvant être obtenue par le procédé selon l'une quelconque des revendications 1 à 17, **caractérisée en ce que** ladite tréhalose-phosphate synthase, l'homologue, ou la partie catalytiquement active de celle-ci est le seul marqueur de sélection transformé dans ladite partie d'une plante ou d'une population de cellules de plante.

19. Utilisation d'un gène homologue de la tréhalose-6-phosphate synthase ou d'un fragment tronqué à l'extrémité N-terminale de celui-ci ou d'un gène hétérologue de la tréhalose-6-phosphate synthase ou d'un fragment de celui-ci ou de l'extrémité N-terminale d'un gène homologue ou hétérologue de la tréhalose-6-phosphate synthase comme marqueur de sélection pour la transformation d'une plante.


### SEQUENCE LISTING

<110> K.U.Leuven R&D
   Thevelein, Johan
   Leyman, Barbara
   Van Dijck, Patrick
   Avonce, Nelson
   Iturriaga, Gabriel
<120> transgenic plant production
<130> K-1932-PCT
<150> UK 0100105.6
   <151> 2000-01-04
<160> 2
<170> PatentIn version 3.1
<210> 1
   <211> 20
   <212> DNA
   <213> Cauliflower mosaic virus
<220>
   <221> misc_feature
   <223> 35S pcr primer
<400> 1
   aagaagacgt tccaaccacg
   20
<210> 2
   <211> 20
   <212> DNA
   <213> Arabidopsis thaliana
<220>
   <221> misc_feature
   <223> AtTPS1 pcr primer
<400> 2
   cgctcagaac aactatggtt
   20